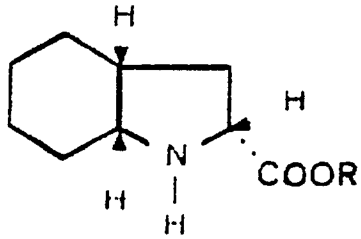

(±) IIa
cis, endo
2S, 3aS, 7aS ou 2R, 3aR, 7aR

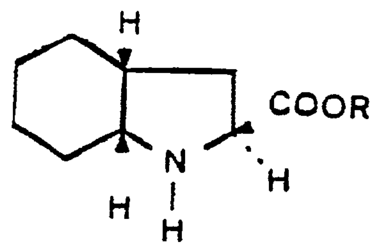

(±) IIb
cis, exo
2S, 3aR, 7aR ou 2R, 3aS, 7aS

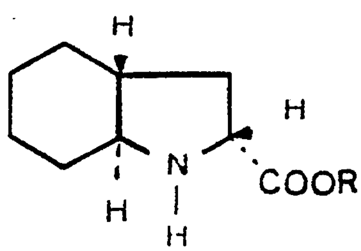

(±) IIc
trans α
2S, 3aS, 7aR ou 2R, 3aR, 7aS

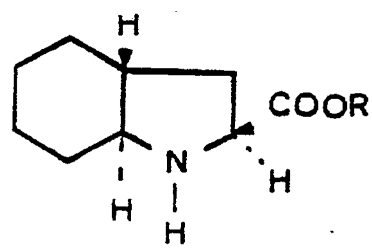

(±) IId
trans β
2R, 3aS, 7aR ou 2S, 3aR, 7aS

La préparation d'un composé de formule (II) peut être réalisée par des techniques bien connues de l'art antérieur (EP 0 037 231, EP 0 084 164, EP 0 115 345, EP 0 173 199 , EP 0 132 580).

Toutefois, l'isomère spécifiquement utilisé dans la synthèse du composé de formule (I), eu égard à sa structure est l'acide perhydroindole carboxylique-2-(2S, 3aS, 7aS) de formule (IIaS):

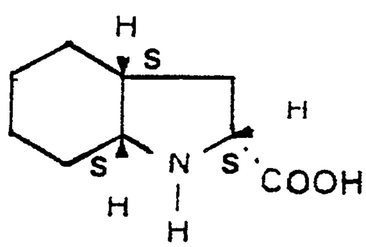

(IIaS)

La préparation de l'acide perhydroindole carboxylique-2-(2S, 3aS, 7aS) peut être réalisée selon des techniques décrites dans l'art antérieur (EP 0 037 231, EP 0 115 345, EP 0 173 199, EP 0 132 580).

Certaines d'entre elles utilisent comme matière première l'acide indole carboxylique-2 qui a l'avantage d'être une matière première facilement disponible et relativement peu onéreuse (EP 0 037 231), lequel est soumis à réduction catalytique sur rhodium pour donner un mélange des deux isomères cis endo de configuration respective (2S, 3aS, 7aS) et (2R, 3aR, 7aR).

Cependant, la séparation de l'isomère (2S, 3aS, 7aS) utile dans la synthèse du dérivé de formule (I) de l'isomère (2R, 3aR, 7aR), nécessite généralement l'emploi de méthodes dont la mise en œuvre est particulièrement laborieuse.

Ainsi, le brevet n° 0 037 231, pour réaliser cette séparation des isomères (2S, 3aS, 7aS) et (2R, 3aR, 7aR) (cis, endo racémique) utilise de nombreuses étapes nécessitant la synthèse du dérivé N-benzoylé, la cristallisation fractionnée du sel du diastéréoisomère avec la S a phényléthylamine, la libération des deux dérivés SSS et RRR N benzoylés, puis l'élimination du groupement benzoyle suivi d'un passage sur colonne échangeuse d'ions et d'une recristallisation.

La demande de brevet européen n° 0 115 345 pour cette même séparation, utilise plusieurs étapes nécessitant l'estérification de la fonction acide carboxylique par l'alcool benzylique, la salification de l'amino ester par la N benzyloxycarbonyl-(S) phénylalanine, la séparation par cristallisation fractionnée de

l'isomère S, S, S, la libération de la fonction aminée optionnellement suivie de la libération du groupement acide carboxylique.

La demanderesse pour synthétiser industriellement le dérivé de formule (I), utilise un procédé original de synthèse de l'acide perhydroindole carboxylique-2-(2S, 3aS, 7aS) présentant également l'avantage d'utiliser comme matière première l'acide indole carboxylique-2, mais qui n'offre pas l'inconvénient de cette laborieuse séparation des deux isomères (2S, 3aS, 7aS) et (2R, 3aR, 7aR) puisque l'acide indole carboxylique-2 est, dans un premier temps réduit en acide indoline carboxylique-2, pour donner un mélange d'acide indoline carboxylique 2R et 2S lesquels sont aisément séparés en une seule étape, par cristallisation fractionnée. L'isomère 2S est ensuite soumis à hydrogénation catalytique pour conduire stéréosélectivement à l'acide perhydroindole carboxylique-2 (2S, 3aS, 7aS).

Plus particulièrement, la synthèse industrielle de l'acide perhydroindole carboxylique-2-(2S, 3aS, 7aS) utilisée pour la synthèse industrielle du dérivé de formule (I) par la demanderesse met en œuvre comme matière première l'acide indole carboxylique-2, ou l'un de ses esters de formule (III):

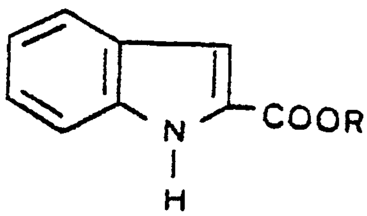

(III)

dans laquelle R représente un atome d'hydrogène, un groupement alcoyle inférieur,

lequel est soumis à réduction par un procédé tel que l'utilisation du couple étain/acide chlorhydrique, préférentiellement en milieu d'alcool aliphatique inférieur, préférentiellement à température ambiante en acide indoline carboxylique-2-(R, S) ou l'un de ses esters de formule (IV):

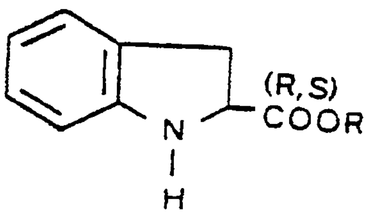

(IV)

dans laquelle R a la même signification que dans la formule (III), qui lorsque R = H, est l'acide indoline carboxylique-2 de formule (V),

qui lorsque R est différent de H est transformé par hydrolyse alcaline en acide indoline carboxylique-2 de formule (V):

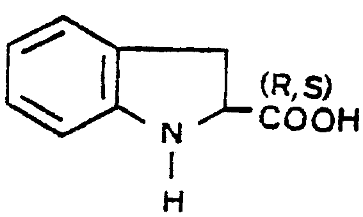

(V)

constitué en fait, par un mélange de deux isomères selon que le carbone porteur du carboxyle se trouve:

– dans la configuration R (isomère R),

– dans la configuration S (isomère S),

mélange dont on isole l'isomère S par addition du dit mélange à une solution de (+) α méthyl benzylamine dans un alcool aliphatique inférieur, pour obtenir un précipité du sel de l'acide indoline carboxylique-2-(S) avec l'α méthyl benzylamine, qui, après filtration, est dissous dans l'eau, la solution obtenue étant alors acidifiée pour permettre la libération de l'acide indoline-2-(S) carboxylique, lequel après filtration et lavage, est soumis à hydrogénation catalytique, sous une pression d'hydrogène comprise entre 10 et 150 bars, préférentiellement choisie entre 20 et 60 bars, en chauffant à une température comprise entre 30 et 100°C, préférentiellement comprise entre 40 et 80°C, en présence de rhodium en mélange à un support tel que le charbon, de façon à permettre l'obtention d'un taux maximal d'acide perhydroindole carboxylique-2-(2S, 3aS, 7aS), celui-ci étant séparé de l'isomère (2S, 3aR, 7aR) obtenu en faible proportion par une cristallisation unique dans le dioxanne en mélange à l'eau.

La seconde matière première utilisée pour la synthèse du composé de formule (I), selon l'invention est le dérivé de formule (VI):

$$\begin{array}{ccccccc} & & (R,S) & & (R,S) & & \\ & & * & & * & & \\ & & & & CH_2CH_2CH_3 & & \\ & & & & | & & \\ HO - OC & - & CH & - NH - & CH & - CO - & OC_2H_5 \\ & & | & & & & \\ & & CH_3 & & & & \end{array} \qquad (VI)$$

Le composé de formule (VI) existe sous forme de quatre stéréoisomères que l'on peut désigner par (R, R); (R, S); (S, R) ou (S, S) selon la configuration des deux carbones dits asymétriques.

Or, le composé de formule (I) le plus actif est celui pour lequel les deux carbones de la chaîne latérale ont tous deux la configuration S.

C'est la raison pour laquelle le procédé de synthèse industrielle du dérivé de formule (I) selon l'invention s'intéresse exclusivement à la synthèse industrielle du composé de formule (VI) dans lequel les deux carbones asymétriques ont tous deux la configuration S.

Aucun procédé de synthèse industrielle spécifique du dérivé de formule (VI) n'a été décrit. On connaît la demande de brevet européen n° 0 117 488, très générale, qui utilise des trifluorométhane sulfonates $\alpha$ carboxylés. Toutefois, la stéréochimie des deux produits de départ doit être rigoureusement choisie afin d'obtenir le diastéréoisomère voulu du produit de formule (I).

Il est par ailleurs connu de l'homme de l'Art que, de façon très générale, pour permettre la séparation de diastéréoisomères obtenus au cours de synthèses où la stéréochimie des matières premières n'est pas préalablement fixée, on a recours à des techniques classiques telles que cristallisation fractionnée ou chromatographie sur colonne de silice.

La demanderesse utilise pour la synthèse industrielle du dérivé de formule (I), un procédé de synthèse industrielle du dérivé de formule (VI) très intéressant car d'une part particulièrement simple à mettre en œuvre, et qui d'autre part permet par un choix judicieux des réactifs catalyseurs et solvants, utilisés l'obtention directe du diastéréoisomère (S, S) avec des rendements industriellement très intéressants.

En outre, la synthèse selon le procédé de l'invention présente l'avantage d'utiliser comme matières premières des dérivés peu coûteux, ce qui, industriellement est d'importance.

Plus particulièrement, le procédé utilisé par la demanderesse pour la synthèse industrielle du dérivé de formule (I) utilise comme produit de départ un dérivé d'acide aminé naturel, de formule (VII):

$$\begin{array}{ccc} & CH_2CH_2CH_3 & \\ & | & \\ H_2N - & CH & - CO - OH \\ & (S) & \end{array} \qquad (VII)$$

dans lequel le carbone asymétrique a la configuration S puisqu'il est bien connu de l'homme de l'Art que le carbone porteur du carboxyle des amino acides naturels a la configuration S (cystéine exceptée), que l'on traite, en présence d'un catalyseur d'estérification acide, et préférentiellement de chlorure de thionyle, par l'éthanol, industriellement disponible à bas prix, pour donner l'ester de formule (VIII):

$$\begin{array}{ccc} & CH_2CH_2CH_3 & \\ & | & \\ H_2N - & CH & - CO - OC_2H_5 \\ & (S) & \end{array} \qquad (VIII)$$

lequel est condensé sous hydrogénation catalytique, sous une pression d'hydrogène comprise entre 10 et 150 bars, préférentiellement choisie entre 20 et 60 bars, préférentiellement à température ambiante, le catalyseur étant choisi parmi le rhodium, le palladium, le platine ou le nickel en mélange à un support tel que le charbon, catalyseur soigneusement sélectionné de façon à orienter la sélectivité de la réaction, permettant ainsi l'obtention d'un taux maximal du diastéréoisomère (S,S), avec l'acide pyruvique $CH_3$-CO-COOH, produit naturel, peu couteux, industriellement disponible, pour conduire directement, après traitement à l'éthanol et simple cristallisation dans l'acétonitrile, refroidissement et filtration, au seul diastéréoisomère (S, S) du dérivé de formule (VI).

L'étape finale de synthèse industrielle du dérivé de formule (I) selon l'invention consiste en la condensation du dérivé de formule (IIaS), dont on a protégé préalablement la fonction acide carboxylique par estérification par l'alcool benzylique en présence d'acide paratoluène sulfonique, pour donner le paratoluène sulfonate de l'ester benzylique de l'acide perhydroindole carboxylique-2-(2S, 3aS, 7aS) de formule (IX):

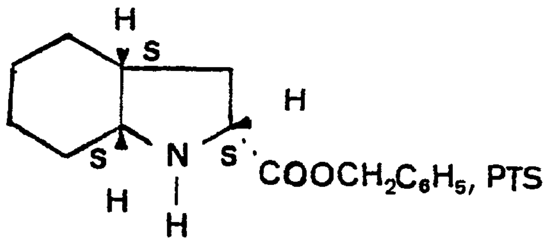

(IX)

lequel est directement condensé au dérivé de formule (VI) en milieu alcalin en présence de dicyclohexylcarbodiimide et d'hydroxy-1 benzotriazole pour conduire au dérivé de formule (X):

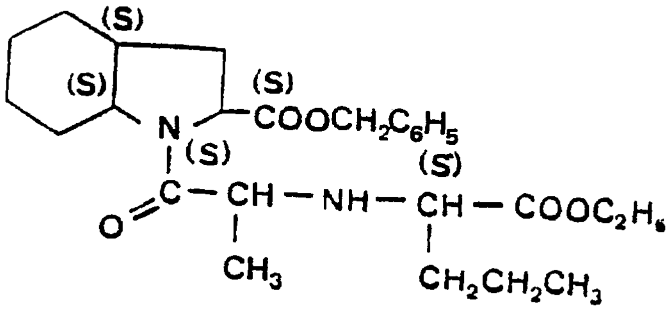

(X)

qui est soumis à déprotection du groupement carboxylique de l'hétérocycle par hydrogénation catalytique sur charbon palladié à température et pression ambiantes, pour conduire au dérivé de formule (I) sous forme de base, laquelle est dissoute dans l'acétate d'éthyle, avant addition de tert.butylamine, le sel ainsi obtenu étant cristallisé par chauffage du milieu réactionnel, filtration à chaud, refroidissement et essorage final.

L'exemple suivant illustre l'invention, mais ne la limite en aucune façon.

EXEMPLE: SYNTHESE INDUSTRIELLE DU SEL DE TERT.BUTYLAMINE DE L'ACIDE [1 (ETHOXYCARBONYL)-1 BUTYLAMINE-(S)-2 PROPIONYL-(S)]-1 OCTHAYDROINDOLE CARBOXYLIQUE-2 (2S, 3aS, 7aS)

STADE 1: ACIDE OCTAHYDROINDOLE CARBOXYLIQUE-2 (2S, 3aS, 7aS)

STADE 1A: Ethoxycarbonyl-2 indole

Chauffer à ébullition 5 kg de carboxy-2 indole, mis en suspension dans l'éthanol en présence d'acide sulfurique durant 8 heures.

Evaporer l'acétate d'éthyle, reprendre la masse cristalline par l'hexane. Après essorage et séchage, on obtient 5,3 kg de cristaux.

Point de fusion: 123–125°C

Microanalyse:

Calculé: C% 69,83 H% 5,86 N% 7,40
Trouvé: C% 69,56 H% 5,74 N% 7,30

Spectrométrie dans l'infra-rouge:

2150 $cm^{-1}$ (NH)
1680 $cm^{-1}$ (acide carboxylique)

STADE 1B: Ethoxycarbonyl-2 (R, S) indoline

Dans un réacteur mettre en suspension 10 kg d'éthoxycarbonyl-2 indoline obtenue précédemment dans 110 litres d'éthanol chlorhydrique. Ajouter ensuite, 20 kg d'étain en grenaille. Maintenir sous agitation à température ambiante pendant 2 jours environ.

Evaporer l'éthanol, reprendre le résidu par l'eau et ajouter 110 litres de toluène. Agiter 20 minutes environ. Alcaliniser par l'ammoniaque. Décanter la phase aqueuse et l'extraire une nouvelle fois par 150 litres de toluène.

Réunir les phases toluéniques et les laver à l'eau. Décanter les phases toluéniques, filtrer. Eliminer l'eau par distillation de l'azéotrope eau toluène Refroidir et faire passer un courant d'HCl gazeux anhydre.

Refroidire. Evaporer, et laver par du toluène pur.

Poids obtenu: 10,11kg

Rendement: 84%

Chromatographie sur couche mince:

Solvant: toluène: 10;
Acétate d'éthyle: 5
Support: silice 60 F 254 MERCK
Révélateur: UV
Rf.: 0,55

STADE 1C: Carboxy-2 (R, S) indoline

2,15 kg d'éthoxy carbonyl-2 (R, S) indoline en solution dans l'éthanol sont saponifiés par 12,5 litres de soude N, sous agitation pendant 24 heures. Après lavage de la solution alcaline, neutraliser par l'acide chlorhydrique concentré. Après essorage, lavage, séchage, on obtient 1,57 kg de cristaux blancs du produit attendu.

Rendement: 86%.

Point de fusion: 188–109°C

Spectrométrie dans l'infra-rouge:

$NH_2^+$: 2500–2000 $cm^{-1}$
$COO^-$: 1620 $cm^{-1}$

STADE 1D: Carboxy-2 (S) indoline

6,05 kg de (R, S) carboxy-2 indoline sont ajoutés à une solution de 4,49 kg de (+) α méthyl benzylamine dans l'éthanol anhydre. On obtient un produit précipité blanc qui, après essorage est digéré dans l'isopropanol chauffé au reflux. Après refroidissement, on essore, lave avec un peu d'isopropanol; les cristaux blancs obtenus sont séchés: 3,68 kg.

Pouvoir rotatoire:

$$[\alpha]_{21}^{D} = -5,3 \ (c = 1\ \%\ \textit{éthanol})$$

La (S) carboxy-2 indoline est préparée avec un rendement quantitatif par dissolution de 1 kg du sel précédent dans 5 litres d'eau et neutralisation par une solution aqueuse d'acide chlorhydrique. Ce précipité est essoré, lavé à l'eau et séché.

STADE 1E: Acide octahydroindole carboxylique-2 (2S, 3aS, 7aS)

Dans une cuve, placer 25 kg d'acide indoline carboxylique-2 (S), précédemment obtenus dans 110 litres de méthanol. Maintenir sous agitation. Dans un mélangeur, charger le catalyseur au rhodium (5% sec).

Dans un hydrogénateur, mettre l'agitation en route, charger la suspension méthanolique d'acide indoline carboxylique-2 (S) en la faisant transiter par le mélangeur et rincer l'ensemble à l'eau. Chauffer à 60°C et mettre en pression d'hydrogène (30 bars).

Filtrer le catalyseur sur filtre monoplaque. Recueillir les jus hydroalcooliques dans un réacteur, et évaporer le méthanol sous vide.

Après concentration, charger 300 kg environ de dioxanne. Porter à ébullition et ajouter de d'eau jusqu'à l'obtention d'une solution. Laisser refroidir. Essorer et sécher.

On obtient 22,3 kg de cristaux.

Rendement: 86,1%.

STADE 2: N-[(S) CARBETHOXY-1 BUTYL] (S) ALANINE

STADE 2A: Chlorhydrate de L-Norvalinate d'éthyle

Dans un réacteur, placer 35 kg de L-Norvaline dans 300 kg environ d'éthanol dénaturé. Introduire lentement et progressivement 60 kg environ de chlorure de thionyle.

Après un quart d'heure d'agitation, chauffer au reflux pendant 3 heures, puis évaporer l'éthanol sous vide.

Reprendre le résidu par 300 litres de cyclohexane, et porter à ébullition. Laisser refroidir, filtrer, laver au cyclohexane et sécher. On obtient 52,9 kg de chlorhydrate de L-Norvalinate d'éthyle, soit un rendement de 97,6%.

Le produit ainsi obtenu est utilisé tel quel au stade suivant.

STADE 2B: N-[(S) carbéthoxy-1 butyl] (S) alanine

Dans une cuve équipée d'un agitateur placer, 45 kg de chlorhydrate de L-Norvalinate d'éthyle obtenu au stade précédent, et environ 110 litres d'eau.

Alcaliniser, verser ensuite très progressivement 23 kg d'acide pyruvique dans la solution précédemment obtenue et agiter le milieu réactionnel pendant 30 minutes.

Placer dans un appareil à hydrogéner du charbon palladié à 5% en suspension dans l'eau et la solution alcaline de L-Norvalinate d'éthyle précédemment obtenue.

Hydrogéner sous pression (30 bars) à température ambiante pendant une journée environ.

Filtrer sous vide, et évaporer le filtrat sous pression réduite, essorer et sécher. Traiter le résidu obtenu par de l'éthanol; éliminer l'insoluble, constitué de chlorure de sodium, par filtration et le rincer à l'éthanol. Réunir les solutions éthanoliques; évaporer l'éthanol sous pression réduite et, cristalliser le résidu dans l'acétonitrile.

On obtient 34,3 kg de N-[(S) carbéthoxy-1 butyl]-(S) alanine, soit un rendement de 63,9%.

STADE 3: SEL DE TERT.BUTYLAMINE DE L'ACIDE {([ETHOXYCARBONYL)-1 BUTYLAMINO-(S)]-2 PROPIONYL-(S)}-1 OCTAHYDROINDOLE CARBOXYLIQUE-2-(2S, 3aS, 7aS)

STADE 3A: Para toluène sulfonate de l'ester benzylique de l'acide octahydroindole carboxylique-2-(2S, 3aS, 7aS)

Dans un réacteur de 30 litres, chauffer à reflux 12,5 kg d'acide perhydroindole carboxylique-2 (2S, 3aS, 7aS), 50 kg d'acide para toluène sulfonique et 14,2 kg d'alcool benzylique et 38,4 kg de toluène en éliminant l'eau formée à l'acide d'un décanteur en continu. Lorsqu'il ne décante plus d'eau, refroidir, essorer le précipité formé, et sécher.

Rendement: 91,3%

STADE 3B: Ester benzylique de l'acide {[(éthoxycarbonyl)-1 butylamino-(S)]-2 propionyl (S)}-1 octahydroindole carboxylique-2 (2S, 3aS, 7aS)

Dans une suspension de 5 kg environ de para toluène sulfonate de l'ester benzylique de l'acide octahydroindole carboxylique-2 (2S, 3aS, 7aS) dans 60 kg environ d`acétate d`éthyle, ajouter 3,5 kg environ de triéthylamine puis 6 kg environ d'hydroxy-1 benzotriazole, 7,5 kg environ de N-[(S) carbéthoxy-1 butyl]-(S) alanine obtenue au stade 2 et 7,0 kg environ de dicyclohexyl carbodiimide.

(19) Europäisches Patentamt
European Patent Office
Office européen des brevets

(11) Numéro de publication: **0 308 341 B1**

(12) # FASCICULE DE BREVET EUROPEEN

(45) Date de publication du fascicule du brevet: **12.12.90**

(51) Int. Cl.⁵: **C07K 5/06**, C07D 209/42, C07C 229/16 // A61K37/02

(21) Numéro de dépôt: **88402339.1**

(22) Date de dépôt: **16.09.88**

(54) **Procédé de synthèse industrielle du périndopril et de ses principaux intermédiaires de synthèse.**

(30) Priorité: **17.09.87 FR 8712896**

(43) Date de publication de la demande: **22.03.89 Bulletin 89/12**

(45) Mention de la délivrance du brevet: **12.12.90 Bulletin 90/50**

(84) Etats contractants désignés: **AT BE CH DE ES FR GB GR IT LI LU NL SE**

(56) Documents cités:
**EP-A- 116 842**
**EP-A- 0 049 658**
**EP-A- 0 088 350**
**EP-A- 0 173 199**

**W.J. HOULIHAN et al.:"Indoles Part One", 1972, page 166, The Chemistry of Heterocyclic Compounds - A Series of Monographs", editeurs A. Weissberger et al.**

(73) Titulaire: **ADIR ET COMPAGNIE, 22, rue Garnier, F-92201 Neuilly sur Seine(FR)**

(72) Inventeur: **Vincent, Michel, 8 allée du Prunier Hardy, F-92220 Bagneux(FR)**
Inventeur: **Baliarda, Jean, 25 avenue Jeanne d'Arc, F-92160 Anthony(FR)**
Inventeur: **Marchand, Bernard, 71 rue Laveau, F-45430 Checy(FR)**
Inventeur: **Remond, Georges, 9 avenue des Etats-Unis, F-78000 Versailles(FR)**

**EP 0 308 341 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne un procédé de synthèse industrielle du perindopril de formule (I):

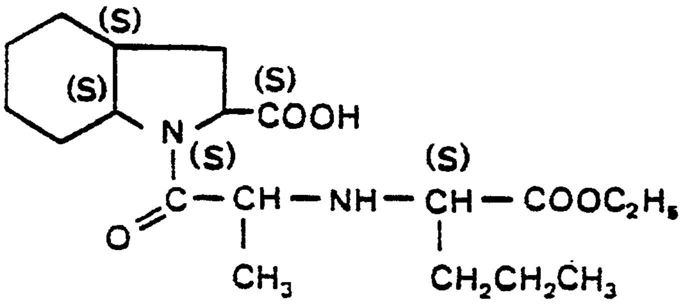

(I)

ou acide {[(éthoxy carbonyl)-1 butylamine-(S)]-2 propionyl-(S)} 1 octahydroindole carboxylique-2 (2S,3aS,7aS) et son sel de tert.butylamine.

Le composé de formule (I) ainsi que ses sels d'addition et plus particulièrement, son sel de tert.butylamine possédent des propriétés pharmacologiques intéressantes. Ils exercent une activité inhibitrice sur certaines enzymes, comme les carboxypeptidases, les enképhalinases ou la kininase II. Ils inhibent notamment, la transformation du décapeptide angiotensine I en l'octapeptide angiotensine II, responsables dans certains cas de l'hypertension artérielle, en agissant sur l'enzyme de conversion.

L'emploi en thérapeutique de ces composés permet donc de réduire ou même supprimer l'activité de ces enzymes responsables de la maladie hypertensive ou de l'insuffisance cardiaque.

En particulier, le composé de formule (I) se distingue des autres inhibiteurs d'enzyme de conversion par l'intensité et la durée de son action.

Le composé de formule (I), sa préparation et son utilisation en thérapeutique ont été décrits dans le brevet européen n° 0 049 658.

Toutefois, la préparation industrielle d'un dérivé tel que le dérivé de formule (I) nécessite une étude approfondie de toutes les étapes réactionnelles, du choix des matières premières, des réactifs, et des solvants permettant d'obtenir les rendements optima.

La demanderesse a découvert un procédé de synthèse du dérivé de formule (I) dans lequel ces conditions ont été réunies par l'utilisation d'un ensemble de techniques et procédés particulièrement intéressants.

En particulier, une des matières premières utilisables pour la préparation des composés de formule (I) est l'acide perhydroindole carboxylique-2, décrit dans la demande de brevet européen n° 0 037 231 ou l'un de ses esters, de formule (II):

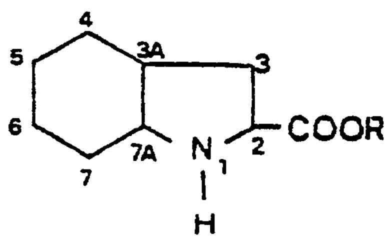

(II)

où R signifie un atome d'hydrogène, un groupement alcoyle inférieur ou benzyle.

Le composé de formule (II) existe sous forme de quatre paires de racémiques:
les deux épimères cis IIa et IIb, les deux épimères trans IIc et IId,

Agiter, en refroidissant légèrement pendant 3 heures environ, puis filtrer la dicyclohexylurée formée par filtration et laver la phase organique par l'eau. La phase organique séchée est évaporée à sec.

Rendement: 92,3%

STADE 3C: Acide {[(éthoxycarbonyl)-1 butylamino-(S)]-2 propionyl (S)}-1 octahydroindole carboxylique-2 (2S, 3aS, 7aS)

Dans un hydrogénateur, dissoudre 14 kg d'ester benzylique de l'acide {[(éthoxycarbonyl)-1 butylamino-(S)]-2 propionyl (S)}-1 octahydroindole carboxylique-2 (2S, 3aS, 7aS), obtenu au stade précédent dans le cyclohexane.

Ajouter le charbon palladié à 5%, et 50 litres d'eau environ. Hydrogéner à température et pression ordinaires jusqu'à absorption du volume théorique d'hydrogène. Filtrer, laver l'insoluble au cyclohexane, décanter la phase organique, et laver la phase aqueuse à nouveau au cyclohexane. Isoler le produit par lyophilisation de la phase aqueuse.

STADE 3D: Sel de tert.butylamine de l'acide {[(éthoxycarbonyl)-1 butylamino è (S)]-2 propionyl (S)}-1 octahydroindole carboxylique-2 (2S, 3aS, 7aS)

Dans un réacteur placer 140 litres environ d'acétate d'éthyle et 10 kg d'acide {[(éthoxycarbonyl)-1 butylamino-(S)]-2 propionyl (S)}-1 de l'acide octahydroindole carboxylique-2 (2S, 3aS, 7aS) obtenu précédemment. Additionner progressivement 2,20 kg environ de tert.butylamine, porter à reflux jusqu'à dissolution totale; filtrer. Refroidir, filtrer et sécher.

Rendement: 95%

**Revendications**

1. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS) de formule (I):

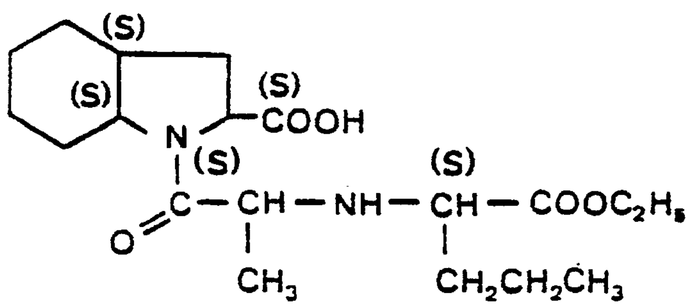

(I)

caractérisé en ce que premièrement l'on soumet:
d'une part, l'acide indoline carboxylique-2 (S) de formule (V),

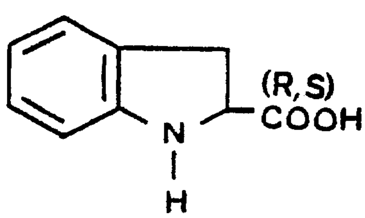

à hydrogénation catalytique, sous une pression d'hydrogène comprise entre 10 et 150 bars, préférentiellement choisie entre 20 et 60 bars, en chauffant à une température comprise entre 30 et 100°C, préférentiellement comprise entre 40 et 80°C, en présence de rhodium en mélange à un support tel que le charbon, de façon à permettre l'obtention d'un taux maximal d'acide perhydroindole carboxylique-2 (2S, 3aS, 7aS) de formule (IIaS)

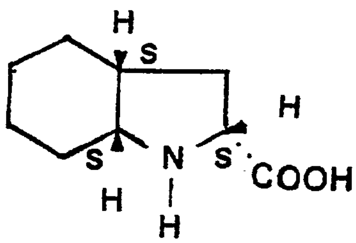

(IIaS)

celui-ci étant séparé de l'isomère (2S, 3aR, 7aR) obtenu en faible proportion par une cristallisation unique dans le dioxanne en mélange à l'eau, la fonction acide carboxylique du dérivé de formule (IIaS) étant alors protégée par estérification par l'alcool benzylique en présence d'acide paratoluène sulfonique pour donner le paratoluène sulfonate de l'ester benzylique de l'acide perhydroindole carboxylique-2 (2S, 3aS, 7aS) de formule (IX):

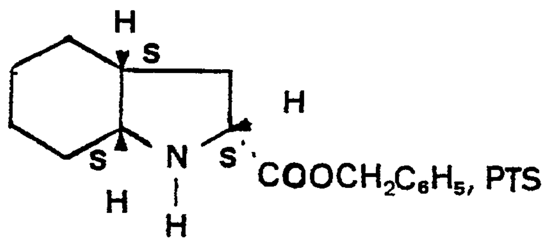

(IX)

d'autre part, la L-Norvaline de formule (VII):

$$\begin{array}{c} \quad CH_2CH_2CH_3 \\ H_2N - \overset{|}{CH} - CO - OH \\ (S) \end{array} \qquad (VII)$$

dans lequel le carbone asymétrique a la configuration S, à estérification par l'éthanol en présence de chlorure de thionyle pour donner l'ester de formule (VIII):

$$\begin{array}{c} \quad CH_2CH_2CH_3 \\ H_2N - \overset{|}{CH} - CO - OC_2H_5 \\ (S) \end{array} \qquad (VIII)$$

lequel est condensé sous hydrogénation catalytique, sous une pression d'hydrogène comprise entre 15 et 60 bars, en présence de charbon palladié, avec l'acide pyruvique $CH_3$-CO-COOH, pour conduire directement, après traitement à l'éthanol, filtration et simple recristallisaton dans l'acétonitrile, refroidissement et filtration, au seul diastéréoisomère (S, S) de la N-[(S) carbéthoxy-1 butyl]-(S) alanine de formule (VI):

$$HO - OC - \underset{CH_3}{\overset{*}{CH}} - NH - \overset{*\ CH_2CH_2CH_3}{CH} - CO - OC_2H_5 \qquad (VI)$$

deuxièmement, en ce que l'on condense le dérivé de formule (VI), avec le dérivé de formule (IX), en milieu alcalin en présence de dicyclohexylcarbodiimide et d'hydroxy-1 benzotriazole pour conduire au dérivé de formule (X):

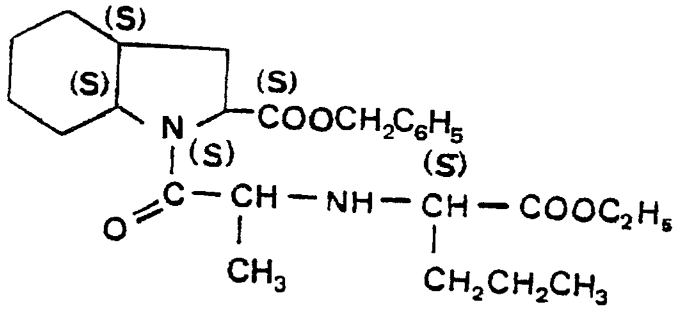

(X)

qui est soumis à déprotection par hydrogénation catalytique sur charbon palladié à température et pression ambiantes, pour conduire au dérivé de formule (I) sous forme de base, laquelle est dissoute dans l'acétate d'éthyle avant addition de tert.butylamine, le sel ainsi obtenu étant cristallisé par chauffage du milieu réactionnel, filtration à chaud, refroidissement et essorage final.

2. Procédé de synthèse industrielle de la N-[(S) carbéthoxy-1 butyl]-(S) alanine de formule (VI):

$$\qquad\qquad * \qquad\quad * \quad CH_2CH_2CH_3$$
$$HO - OC - CH - NH - CH - CO - OC_2H_5 \qquad (VI)$$
$$CH_3$$

dans lequel les deux carbones asymétriques ont la configuration S, utile dans la synthèse du sel de tert.butylamine de l'acide {[(éthoxycarbonyl)-1 butylamino-(S)]-2 propionyl (S)}-1 de l'acide octahydroindole carboxylique-2 (2S, 3aS, 7aS), caractérisé en ce que l'on soumet la L-Norvaline de formule (VII):

$$CH_2CH_2CH_3$$
$$H_2N - CH - CO - OH \qquad (VII)$$
$$(S)$$

dans lequel le carbone asymétrique a la configuration S, à estérification par l'éthanol en présence de chlorure de thionyle, pour donner l'ester de formule (VIII):

$$CH_2CH_2CH_3$$
$$H_2N - CH - CO - OC_2H_5 \qquad (VIII)$$
$$(S)$$

lequel est condensé sous hydrogénation catalytique, sous une pression d'hydrogène comprise entre 15 et 60 bars, en présence de charbon palladié, avec l'acide pyruvique CH3-CO-COOH, pour conduire directement, après traitement à l'éthanol, filtration et simple recristallisation dans l'acétonitrile, refroidissement et filtration, au seul diastéréoisomère (S, S) du dérivé de formule (VI).

3. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon la revendication 1, caractérisé en ce que la réduction de l'acide indoline carboxylique-2 (2S) s'effectue en utilisant le charbon rhodié à 5% comme catalyseur.

4. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon l'une des revendications 1 ou 3, caractérisé en ce que la recristallisation ayant pour but d'isoler l'acide perhydroindole carboxylique-2 (2S, 3aS, 7aS) à partir du mélange réactionnel obtenu après réduction catalytique de l'acide indoline carboxylique-2 (S) s'effectue en utilisant comme solvant le mélange dioxanne-eau.

5. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon l'une des revendications 1 ou 2, caractérisé en ce que la condensation de l'acide pyruvique avec l'ester éthylique de la L-Norvaline sous hydrogénation, s'effectue sous une pression d'hydrogène comprise entre 20 et 60 bars.

6. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon l'une des revendications 1, 2 ou 5, caractérisé en ce que la condensation de l'acide pyruvique avec l'ester éthylique de la L-Norvaline sous hydrogénation, s'effectue à température ordinaire.

7. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon l'une des revendications

1, 2, 5 ou 6, caractérisé en ce que la condensation de l'acide pyruvique avec l'ester éthylique de la L-Norvaline sous hydrogénation s'effectue dans l'eau.

8. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon l'une des revendications 1, 3, 4, 5, 6 ou 7, caractérisé en ce que la protection de la fonction acide carboxylique de l'acide perhydroindole carboxylique-2 (2S, 3aS, 7aS) préalable à la condensation de ce dernier avec la N-[(S) carbétoxy-1 butyl]-(S) alanine de formule (VIII) s'effectue par l'alcool benzylique.

9. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon la revendication 1 ou 3 à 8, caractérisé en ce que la protection de la fonction acide carboxylique de l'acide perhydroindole carboxylique-2 (2S, 3aS, 7aS) préalable à la condensation de ce dernier avec la N-[(S) carbétoxy-1 butyl]-(S) alanine de formule (VIII) s'effectue par l'alcool benzylique, en utilisant comme catalyseur l'acide para toluène sulfonique.

10. Procédé de synthèse industrielle du sel de tert.butylamine de l'acide {[(éthoxycarbonyl)-1 butylamino-(S)]-2 propionyl (S)}-1 octahydroindole carboxylique-2 (2S, 3aS, 7aS), selon la revendication 1, ou 3 à 9, caractérisé en ce que la déprotection de la fonction acide carboxylique de l'acide perhydroindole carboxylique - 2 (2S, 3aS, 7aS) faisant suite à la condensation de ce dernier avec la N-[(S) carbéthoxy-1 butyl]-(S) alanine de formule (VIII) s'effectue par hydrogénation catalytique à température et pression ambiantes, en présence de charbon palladié.

**Patentansprüche**

1. Verfahren zur technischen Synthese des tert.-Butylaminsalzes der 1{2-[1-(Ethoxycarbonyl)-(S)-butylamino]-(S)-propionyl}-(2S,3aS,7aS)-octahydroindol-2-carbonsäure der Formel (I)

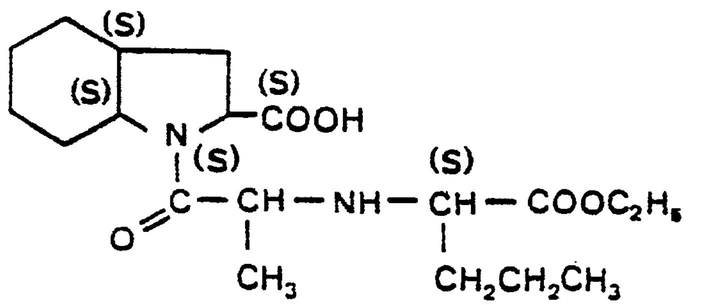

(I)

dadurch gekennzeichnet, daß man <u>erstens einerseits</u> (S)-Indol-2-carbonsäure der Formel (V)

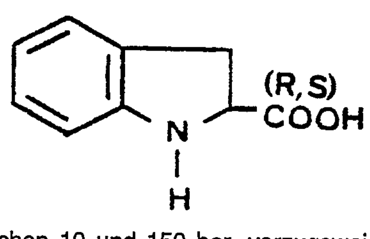

unter einem Wasserstoffdruck zwischen 10 und 150 bar, vorzugsweise zwischen 20 und 60 bar, durch Erhitzen auf eine Temperatur zwischen 30 und 100°C, vorzugsweise zwischen 40 und 80°C, in Gegenwart von Rhodium in Mischung mit einem Trägermaterial, wie Kohlenstoff, katalytisch hydriert derart, daß man (2S, 3aS, 7aS)-Perhydroindol-2-carbonsäure der Formel (IIaS)

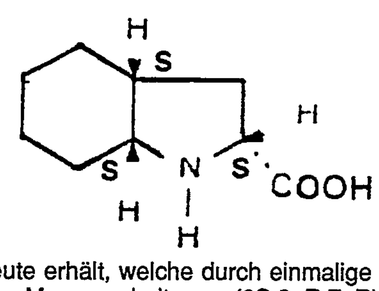

(IIaS)

in maximaler Ausbeute erhält, welche durch einmalige Kristallisation aus mit Wasser vermischtem Dioxan von dem in geringer Menge erhaltenen (2S,3aR,7aR)-Isomeren abgetrennt wird, die Carbonsäuregruppe des Derivats der Formel (IIaS) anschliessend durch Verestern mit Benzylalkohol in Gegenwart von

p-Toluolsulfonsäure schützt unter Bildung des p-Toluolsulfonats des Benzylesters der (2S,3aS,7aS)-Perhydroindol-2-carbonsäure der Formel (IV)

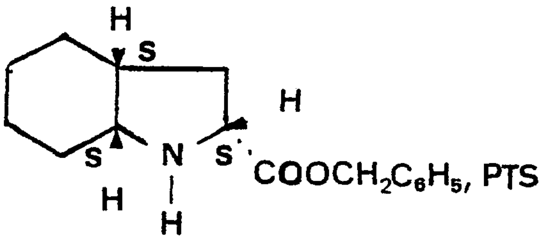

(IX)

andererseits L-Norvalin der Formel (VII)

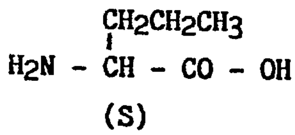

(VII)

dessen asymmetrisches Kohlenstoffatom in der S-Konfiguration vorliegt, mit Ethanol in Gegenwart von Thionylchlorid verestert unter Bildung des Esters der Formel (VIII)

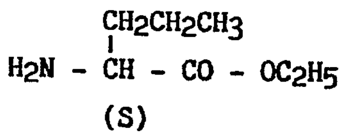

(VIII)

welcher unter katalytischer Hydrierung bei einem Wasserstoffdruck zwischen 15 und 60 bar in Gegenwart von Palladium-auf-Kohlenstoff mit Brenztraubensäure $CH_3$-CO-COOH kondensiert wird, so daß man direkt nach der Behandlung mit Ethanol, Filtration und einfache Umkristallisation aus Acetonitril, Abkühlen und Filtration das einzige (S,S)-Diastereoisomere des N-[(S)-1-Carbethoxy-butyl]-(S)-alanins der Formel (VI)

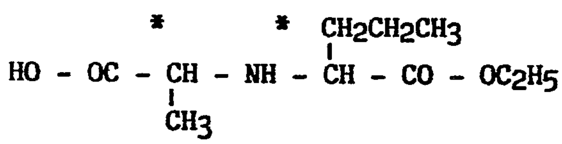

(VI)

erhält,

zweitens das Derivat der Formel (VI) in alkalischem Medium und in Gegenwart von Dicyclohexylcarbodiimid und 1-Hydroxybenzotriazol mit dem Derivat der Formel (IX) kondensiert unter Bildung des Derivats der Formel (X)

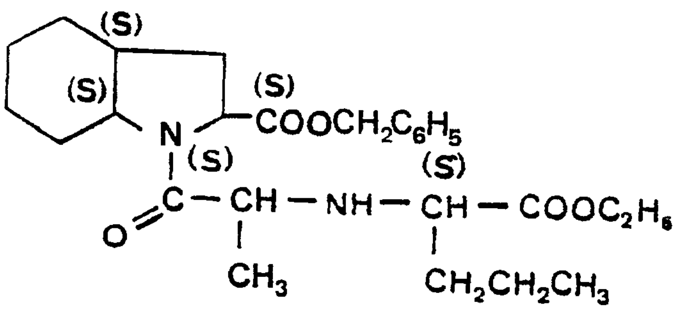

(X)

welches durch katalytische Hydrierung über Palladium-auf-Kohlenstoff bei Raumtemperatur und Umgebungsdruck von den Schutzgruppen befreit wird, so daß man das Derivat der Formel (1) in Form der Base erhält, welche in Ethylacetat gelöst wird, bevor man tert.-Butylamin zugibt und das in dieser Weise erhaltene Salz durch Erhitzen in dem Reaktionsmedium, Abfiltrieren in der Wärme und Abkühlen kristallisiert und schließlich absaugt.

2. Verfahren zur technischen Synthese von N-[(S)-1-Carbethoxy-butyl]-(S)-alanin der Formel (VI)

$$HO - OC - \underset{\displaystyle CH_3}{\overset{*}{CH}} - NH - \overset{*\ CH_2CH_2CH_3}{CH} - CO - OC_2H_5 \qquad (VI)$$

dessen beide asymmetrischen Kohlenstoffatome in der S-Konfiguration vorliegen, welches zur Synthese des tert.-Butylaminsalzes der 1-{2-[1-(Ethoxycarbonyl)-(S)-butylaminol]-(S)-propionyl}-(2S,3aS, 7aS)-octahydroindol-2-carbonsäure geeignet ist, dadurch gekennzeichnet, daß man L-Norvalin der Formel (VII)

$$H_2N - \underset{(S)}{\overset{CH_2CH_2CH_3}{CH}} - CO - OH \qquad (VII)$$

in der das asymmetrische Kohlenstoffatom in der S-Konfiguration vorliegt, mit Ethanol in Gegenwart von Thionylchlorid verestert unter Bildung des Esters der Formel (VIII)

$$H_2N - \underset{(S)}{\overset{CH_2CH_2CH_3}{CH}} - CO - OC_2H_5 \qquad (VIII)$$

welchen man unter katalytischer Hydrierung bei einem Wasserstoffdruck zwischen 15 und 60 bar in Gegenwart von Palladium-auf-Kohlenstoff mit Brenztraubensäure $CH_3$-CO-COOH kondensiert, so daß man direkt nach der Behandlung mit Ethanol, Filtrieren und einfache Umkristallisation aus Acetonitril, Abkühlen und Filtration das einzige (S, S)-Diastereoisomere des Derivats der Formel (VI) erhält.

3. Verfahren zur technischen Synthese des tert.-Butylaminsalzes der 1{2-[1-(Ethoxycarbonyl)-S)-butylamino]-(S)-propionyl}-(2S,3aS,7aS)-octahydroindol-2-carbonsäure der Formel (I) nach Anspruch 1, dadurch gekennzeichnet, daß man die Reduktion der (2S)-Indolin-2-carbonsäure unter Verwendung von 5% Rhodium-auf-Kohlenstoff als Katalysator durchführt.

4. Verfahren zur technischen Synthese des tert.Butylaminsalzes der 1{2-[1-(Ethoxycarbonyl)-S)-butylamino]-(S)-propionyl}-(2S,3aS,7aS)-octahydroindol-2-carbonsäure der Formel (I) nach einem der Ansprüche 1 oder 3, dadurch gekennzeichnet, daß die Umkristallisation zur Isolierung der (2S,3aS, 7aS)-Perhydroindol-2-carbonsäure ausgehend von der bei der katalytischen Reduktion der (S)-Indolin-2-carbonsäure erhaltenen Reaktionsmischung unter Verwendung einer Dioxan/Wasser-Mischung als Lösungsmittel durchgeführt wird.

5. Verfahren zur technischen Synthese des tert.-Butylaminsalzes der 1{2-[1(Ethoxycarbonyl)-S)-butylamino]-(S)-propionyl}-(2S,3aS,7aS)-octahydroindol-2-carbonsäure der Formel (I) nach einem der Ansprüche 1 oder 2, dadurch gekennzeichnet, daß die Kondensation der Brenztraubensäure mit dem Ethylester des L-Norvalins unter Hydrieren bei einem Wasserstoffdruck zwischen 20 und 60 bar durchgeführt wird.

6. Verfahren zur technischen Synthese des tert.-Butylaminsalzes der 1{2-[1(Ethoxycarbonyl)-S)-butylamino]-(S)-propionyl}-(2S,3aS,7aS)-octahydroindol-2-carbonsäure der Formel (I) nach den Ansprüchen 1, 2 oder 5, dadurch gekennzeichnet, daß die Kondensation der Brenztraubensäure mit dem Ethylester des L-Norvalins unter Hydrieren bei Umgebungstemperatur bewirkt wird.

7. Verfahren zur technischen Synthese des tert.-Butylaminsalzes der 1{2-[1(Ethoxycarbonyl)-S) -butylamino]-(S)-propionyl}-(2S, 3aS, 7aS)-octahydroindol-2-carbonsäure der Formel (I) nach den Ansprüchen 1, 2, 5 oder 6, dadurch gekennzeichnet, daß die Kondensation der Brenztraubensäure mit dem Ethylester des L-Norvalins unter Hydrierung in Wasser durchgeführt wird.

8. Verfahren zur technischen Synthese des tert.-Butylaminsalzes der 1{2-[1(Ethoxycarbonyl)-(S)-butylamino]-(S)-propionyl}-(2S, 3aS, 7aS)-octahydroindol-2-carbonsäure der Formel (I) nach den Ansprüchen 1, 3, 4, 5, 6 oder 7, dadurch gekennzeichnet, daß der Schutz der Carbonsäuregruppe der (2S,3aS, 7aS)-Perhydroindol-2-carbonsäure vor der Kondensation dieser Säure mit N-[(S)-1-Carbethoxy-butyl]-(S)-alanin der Formel (VIII) mit Benzylalkohol bewirkt wird.

9. Verfahren zur technischen Synthese des tert.-Butylaminsalzes der 1{2-[1(Ethoxycarbonyl)-(S)-butylamino]-(S)-propionyl}-(2S, 3aS, 7aS)-octahydroindol-2-carbonsäure der Formel (I) nach den Ansprüchen 1 oder 3 bis 8, dadurch gekennzeichnet, daß der Schutz der Carbonsäuregruppe der (2S,3aS, 7aS)-Perhydroindol-2-carbonsäure vor der Kondensation dieser Säure mit N-[(S)-1-Carbethoxy-butyl]-(S)-alanin der Formel (VIII) mit Benzylalkohol unter Verwendung von p-Toluolsulfonsäure als Katalysator bewirkt wird.

10. Verfahren zur technischen Synthese des tert.-Butylaminsalzes der 1{2-[1(Ethoxycarbonyl)-S)-butylamino]-(S)-propionyl}-(2S,3aS,7aS)-octahydroindol-2-carbonsäure der Formel (I) nach den Ansprüchen 1, 3 oder 9, dadurch gekennzeichnet, daß die Abspaltung der Schutzgruppe von der Carbonsäuregruppe der (2S,3aS,7aS)-Perhydroindol-2-carbonsäure im Anschluß an die Kondensation diese Säure mit N-[(S)-1-Carbethoxy-butyl]-(S)-alanin der Formel (VIII) durch katalytische Hydrierung bei Raumtemperatur und Umgebungsdruck in Gegenwart von Palladium-auf-Kohlenstoff bewirkt wird.

**Claims**

1. Process for the industrial synthesis of the tert.-butylamine salt of (2S,3aS,7aS)-1-{2-[1-(ethoxycarbonyl)(S)-butylamino]-(S)-propionyl}octahydroindole-2-carboxylic acid of formula (I):

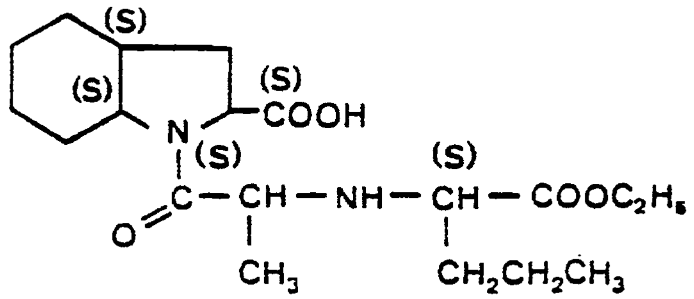

(I)

characterized in that firstly:
on the one hand (S)-2-carboxyindoline of formula (V):

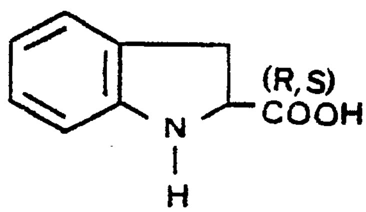

is subjected to catalytic hydrogenation, under a hydrogen pressure of between 10 and 150 bars, preferably chosen between 20 and 60 bars, with heating to a temperature of between 30 and 100°C, preferably between 40 and 80°C, in the presence of rhodium mixed with a support such as charcoal, so as to make it possible to obtain a maximum proportion of (2S,3aS,7aS)-2-carboxyperhydroindole of formula (IIaS):

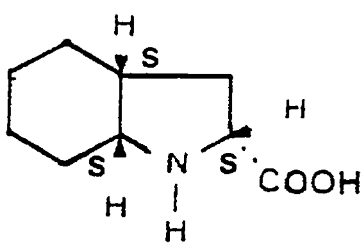

(IIaS)

the latter being separated from the (2S,3aR,7aR) isomer obtained in a low proportion by a single crystallization from dioxane, mixed with water, the carboxylic acid functional group of the derivative of formula (IIaS) being then protected by esterification with benzyl alcohol para-toluenesulphonic acid in the presence of the para-toluenesulphonate of the benzyl to give ester of (2S,3aS,7aS)-2-carboxyperhydroindole of formula (IX):

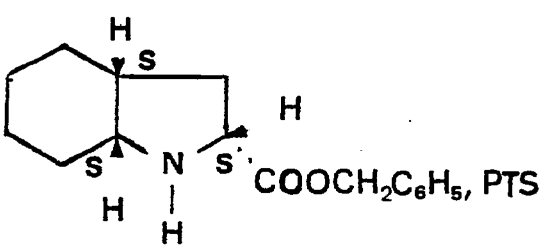

(IX)

on the other hand, L-norvaline of formula (VII):

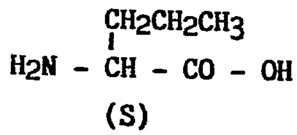 (VII)

in which the asymmetric carbon has the S configuration, is subjected to esterification with ethanol in the presence of thionyl chloride, to give the ester of formula (VIII):

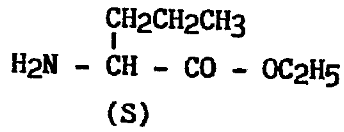 (VIII)

which is condensed under catalytic hydrogenation, under a hydrogen pressure of between 15 and 60 bars, in the presence of palladized charcoal, with pyruvic acid $CH_3$-CO-COOH, to lead directly, after treatment with ethanol, filtration and a single recrystallization from acetonitrile, cooling and filtration, solely to the (S,S) diastereoisomer of N-[(S)-1-carbethoxybutyl]-(S)-alanine of formula (VI):

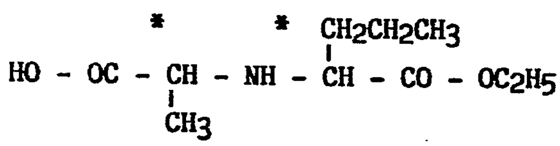 (VI)

secondly, in that the derivative of formula (VI) is condensed with the derivative of formula (IX) in an alkaline medium in the presence of dicyclohexylcarbodiimide and 1-hydroxybenzotriazole to lead to the derivative of formula (X):

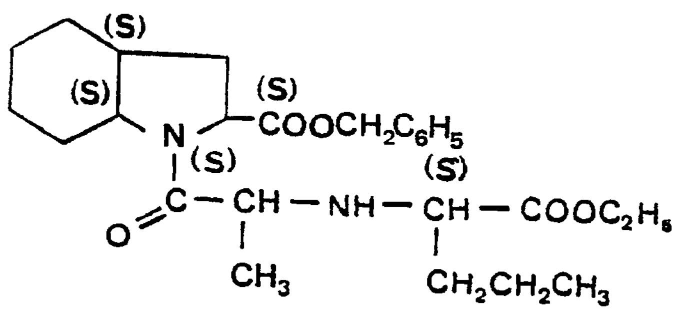 (X)

which is subjected to deprotection by catalytic hydrogenation on palladized charcoal at ambient temperature and pressure, to lead to the derivative of formula (I) in the form of a base, which base is dissolved in ethyl acetate before the addition of tert-butylamine, the salt thus obtained being crystallized by heating the reaction mixture, filtering hot, cooling and finally filtering off.

2. Process for the industrial synthesis of N-[(S)l-1-carbethoxybutyl]-(S)-alanine of formula (VI):

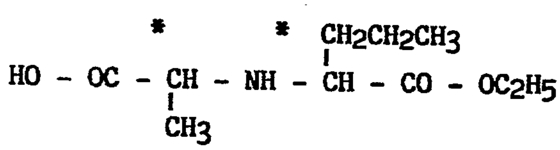 (VI)

in which the two asymmetric carbons have the S configuration, which can be used in the synthesis of the tert-butylamine salt of (2S,3aS,7aS)-1-{2-[1-(ethoxycarbonyl)-(S)-butylamino]-(S)-propionyl}octahydroindole-2-carboxylic acid, characterized in that L-norvaline of formula (VII):

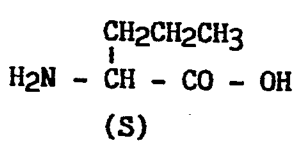 (VII)

in which the asymmetric carbon has the S configuration, is subjected to esterification with ethanol in the presence of thionyl chloride, to give the ester of formula (VIII):

$$\begin{array}{c} \quad CH_2CH_2CH_3 \\ \quad | \\ H_2N - CH - CO - OC_2H_5 \\ (S) \end{array} \qquad (VIII)$$

which is condensed under catalytic hydrogenation, under a hydrogen pressure of between 15 and 60 bars, in the presence of palladized charcoal, with pyruvic acid $CH_3$-CO-COOH, to lead directly, after treatment with ethanol, filtration and a single recrystallization from acetonitrile, cooling and filtration, solely to the (S,S) diastereoisomer of the derivative of formula (VI).

3. Process for the industrial synthesis of the tert.-butylamine salt of (2S,3aS,7aS)-1-{2-[1-(ethoxycarbonyl)-(S)-butylamino]-(S)-propionyl}octahydroindole-2-carboxylic acid, according to Claim 1, characterized in that the reduction of (2S)-2-carboxyindoline is performed using 5% rhodized charcoal as catalyst.

4. Process for the industrial synthesis of the tert-butylamine salt of (2S,3aS,7aS)-1-{2-[1-(ethoxycarbonyl)-(S)-butylamino]-(S)-propionyl}octahydroindole-2-carboxylic acid, according to either of Claims 1 and 3, characterized in that the recrystallization for the purpose of isolating (2S,3aS,7aS)-2-carboxyperhydroindole from the reaction mixture obtained after catalytic reduction of (S)-2-carboxyindoline is performed using a dioxane-water mixture as solvent.

5. Process for the industrial synthesis of the tert-butylamine salt of (2S,3aS,7aS)-1-{2-[1-ethoxycarbonyl)-(S)-butylamino]-(S)-propionyl}octahydroindole-2-carboxylic acid, according to either of Claims 1 and 2, characterized in that the condensation of pyruvic acid with the ethyl ester of L-norvaline under hydrogenation is performed at a hydrogen pressure of between 20 and 60 bars.

6. Process for the industrial synthesis of the tertbutylamine salt of (2S,3aS,7aS)-1-{2-[1-ethoxycarbonyl)(S)-butylamino]-(S)-propionyl}octahydroindole-2-carboxylic acid, according to one of Claims 1, 2 and 5, characterized in that the condensation of pyruvic acid with the ethyl ester of L-norvaline under hydrogenation is performed at ordinary temperature.

7. Process for the industrial synthesis of the tert-butylamine salt of (2S,3aS,7aS)-1-{2-[1-ethoxycarbonyl)-(S)-butylamino]-(S)-propionyl}octahydroindole-2-carboxylic acid, according to one of Claims 1, 2, 5 and 6, characterized in that the condensation of pyruvic acid with the ethyl ester of L-norvaline under hydrogenation is performed in water.

8. Process for the industrial synthesis of the tert-butylamine salt of (2S,3aS,7aS)-1-{2-[1-ethoxycarbonyl)-(S)-butylamino]-(S)-propionyl}octahydroindole-2-carboxylic acid, according to one of Claims 1, 3, 4, 5, 6 and 7, characterized in that the protection of the carboxylic acid functional group of (2S,3aS,7aS)-2-carboxyperhydroindole preceding the condensation of the latter with N-[(S)-1-carbethoxybutyl]-(S)-alanine of formula (VIII), is performed by means of benzyl alcohol.

9. Process for the industrial synthesis of the tert-butylamine salt of (2S,3aS,7aS)-1-{2-[1-ethoxycarbonyl)-(S)-butylamino]-(S)-propionyl}octahydroindole-2-carboxylic acid, according to Claim 1 or 3 to 8, characterized in that the protection of the carboxylic acid functional group of (2S,3aS,7aS)-2-carboxyperhydroindole preceding the condensation of the latter with N-[(S)-1-carbethoxybutyl]-(S)-alanine of formula (VIII) is performed by means of benzyl alcohol, using para-toluenesulphonic acid as catalyst.

10. Process for the industrial synthesis of the tert-butylamine salt of (2S,3aS,7aS)-1-{2-[1-ethoxycarbonyl)-(S)-butylamino]-(S)-propionyl}octahydroindole-2-carboxylic acid, according to Claim 1, or 3 to 9, characterized in that the deprotection of the carboxylic acid functional group of (2S,3aS,7aS)-2-carboxyperhydroindole following the condensation of the latter with N-[(S)-1-carbethoxybutyl]-(S)-alanine of formula (VIII) is performed by catalytic hydrogenation at ambient temperature and pressure, in the presence of palladized charcoal.